Europäisches Patentamt

(19) European Patent Office

Office européen des brevets

(11) Publication number: **0 005 939**
A1

# (12) EUROPEAN PATENT APPLICATION

(21) Application number: 79300854.1

(22) Date of filing: 17.05.79

(51) Int. Cl.²: **A 61 M 5/28**

(30) Priority: 31.05.78 US 911203

(43) Date of publication of application:
12.12.79 Bulletin 79/25

(84) Designated Contracting States:
AT BE CH DE FR GB IT NL SE

(71) Applicant: Kopfer, Rudolph J.
10 Ridgecrest Road
Kentfield California 94904(US)

(72) Inventor: Kopfer, Rudolph J.
10 Ridgecrest Road
Kentfield California 94904(US)

(74) Representative: MacGregor, Gordon et al,
ERIC POTTER & CLARKSON 14 Oxford Street
Nottingham, NG1 5BP(GB)

(54) Compartmental syringe.

(57) A compartmental syringe having an outer casing (1) open at one end and provided with a normally closed outlet (19) at the opposite end, a cylinder (6) reciprocable within the casing in which latter works a plunger; the cylinder being spaced from the inner periphery of the casing to form an outer chamber (13) and the space within the cylinder forming an inner chamber (14) for containing materials to be mixed to form a composition for injection. A nozzle (12) on the cylinder extends through a sealing gland (17) and can be withdrawn therefrom to communicate the outer chamber with the inner chamber so that reciprocation of the plunger mixes the materials. The outlet (19) is opened after mixing and attached to a needle for injection.

EP 0 005 939 A1

./...

FIG.1

1

## COMPARTMENTAL SYRINGE

This invention relates to a compartmental syringe.

Various types of syringes are known at present with means for mixing components or ingredients. Some are too complex for mass production and others do not accomplish a hermetic seal of the ingredients before intermixture and do not preserve them in a sterile state. Some have to be assembled before use, some are too expensive to be disposable and some are difficult to work without error.

The present invention solves the problems of how to design a compartmental syringe wherein the pressures in the outer chamber and in the inner chamber are so regulated during operation as to prevent excessive pressure in the outer chamber and facilitate discharge from the inner chamber without blockage, and allow intermixture without disassembling the device or without admitting any gas or air from the atmosphere.

Another object of the invention is to provide a device which has the ability to lyophilize in the inner chamber or in the outer chamber and which also has the ability to store medicinal ingredients under positive or negative pressure environment; and wherein agitation or mixing of diluent and powder ingredients can be rapidly and efficiently accomplished.

Another object of the invention is to provide a combination syringe and mixer wherein the operative sealed portion of the cylinder surface is protected against contamination while manipulated for mixing and then manipulated for positioning for injection.

Another object of the invention is to provide a combined syringe and mixer capable of separately containing the ingredients to be mixed, and which is positively manipulatable for intermixing by the operation of a plunger; and which imparts a swirling pulsating action to the ingredients expelled from an inner chamber for thorough mixing with the contents of an outer chamber, and in which the parts in contact with the ingredients and with the mixture are maintained in sterile condition.

Another object of the invention is to provide a combined syringe and mixer which can be used easily without inservice training, and which can be manufactured at low cost, and which is capable of accurate release of ingredients, and the principals of which can be incorporated into syringes of any size.

Reference is now made to the accompanying drawings wherein:-

  Figure 1 is a longitudinal sectional view of a portion of a compartment syringe. According to the invention, the syringe containing injectable material

  Figure 2 is a fragmental view showing a manipulating portion of the syringe and a stem of a plunger of the syringe.

  Figure 3 is a fragmental view showing the discharge end of a nozzle of the syringe, showing mixing grooves therein

  Figure 4 is an end elevation showing the discharge end of the nozzle and showing the grooves in the end.

  Figure 5 is a perspective view of a gland for the nozzle showing spiral radial grooves.

Figure 6 is a longitudinal sectional view of the discharge portion of a modified form of the syringe.

Figure 7 is a fragmental partial longitudinal sectional view of the handle portion of the modified form of the syringe.

Figure 8 is a cross-sectional view of the plunger in an inner cylinder of the modified syringe, taken on lines 8 - 8 of Figure 7.

Figure 9 is a fragmental view of another modified form of the syringe in storage position.

Figure 10 is a fragmental view of the plunger end of the syringe of Figure 9, with the plunger in outermost position.

Figure 11 is a fragmental detailed view of the plunger of the syringe of Figure 10

Figure 12 is a cross-sectional view of a nozzle gland with a split valve.

Figure 13 is an elevation of the discharge end of the nozzle gland of Figure 12.

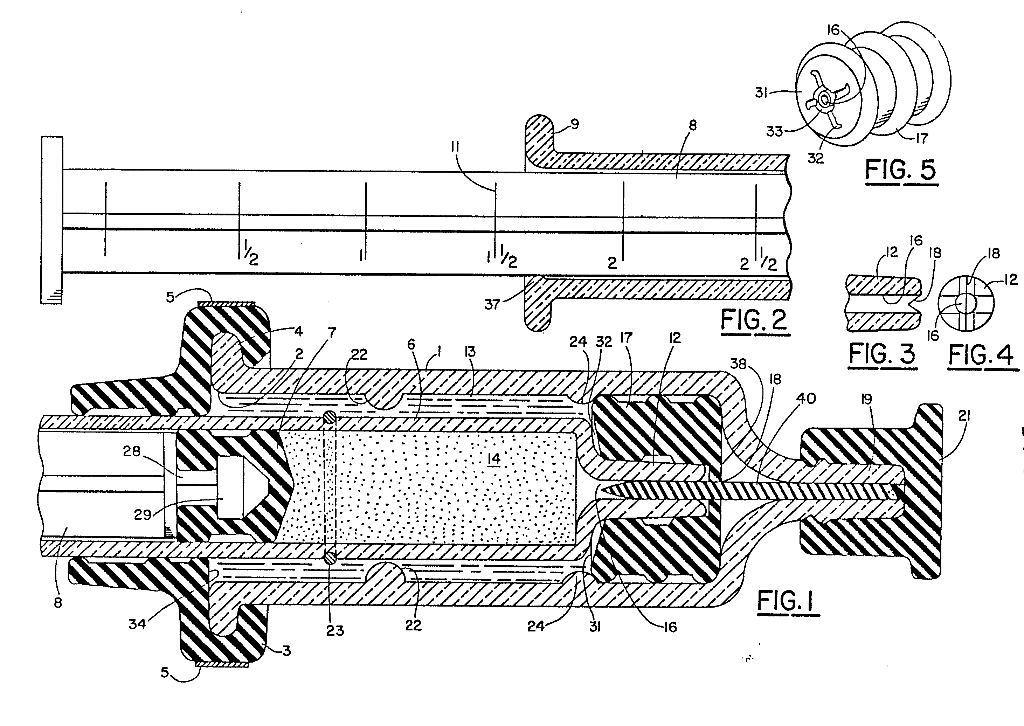

The syringe shown in Figures 1 to 5 comprises an outer casing 1 having an open end 2 having an out-turned end flange 4. An outer seal 3 in the form of a collar is mounted on the flange and is tightly pressed on by a band 5. The other end of the outer casing is normally closed and is adapted to accommodate a syringe needle in a manner to be hereinafter described.

A cylinder 6 extends through and sealing engages with the outer seal 3 and a deformable plunger 7 works in the cylinder. A stem 8 of the plunger 7 extends beyond the outer end 9 of the cylinder 6. The stem 8 has graduations 11 thereon corresponding to the volume of the cavity of the cylinder in various positions of the plunger 7. The inner end of the cylinder 6 is an elongate nozzle 12. The outer diameter of the cylinder 6 is smaller than the inside diameter of the outer casing 1 and thereby defines an annular outer chamber 13 with the outer casing. The cavity in the cylinder may be regarded as an inner chamber 14. The outer casing 1 has guide projections 22 to assist in guiding the cylinder linearly. The outer chamber 13 and the inner chamber 14 can communicate through the passage 16 of the nozzle 12 but this passage is usually closed. The nozzle 12 is sealed by a nozzle gland 17 which, in the form shown in Figure 1 surrounds the nozzle 16 and engages with the inner periphery of the outer casing 1. The gland is made of resilient material and is held in position by projections 24 on the inner periphery of the casing 1 and by the shaping of the outer casing adjacent the closed end. Hence outward movement of the cylinder withdraws the nozzle 12 from the gland 17. This enlarges the outer chamber because the nozzle is of smaller outer diameter than the remainder of the cylinder 6.

On the face 31 of the nozzle gland 17 facing the cylinder 6 are a plurality of grooves 32 radiating from a central indent 33. The limit of outward movement of the cylinder 6 is such that the end of the tip of the cylinder nozzle 12 is spaced at a very short clearance from the adjacent face 31 of the nozzle gland 17, whereby material expelled through the nozzle 12 from the cylinder is deflected through the grooves 32 and acquires a swirling action to assist in mixing the material with the contents of the outer chamber.

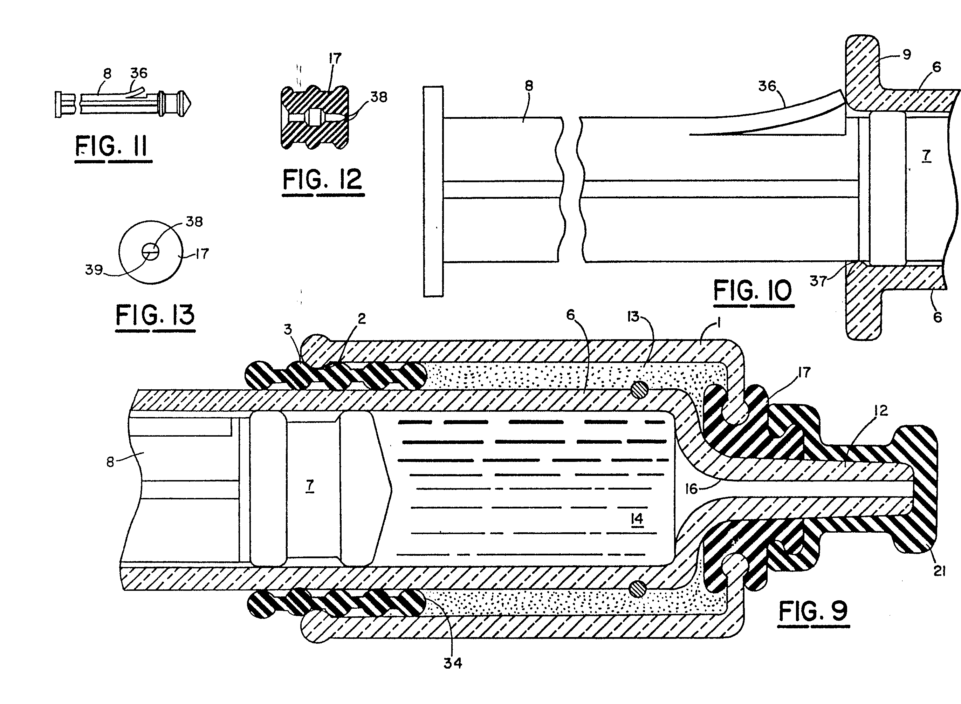

The nozzle gland 17 may be provided with a slit valve consisting of a pair of weakened resilient projections 38 at the discharge face of the nozzle 17 oppositely to form a closed slit 39, as shown in Figures 12 and 13, which are parted only under a predetermined pressure to open the slit 39.

5

The cap 21 has a plug 40 extended from the base of the hollow hub of the cap 21, which plugs the passage of the casing nozzle 19 to prevent escapement of the material from the casing during storage. The plug 40 also plugs the cylinder nozzle 12.

In the form shown in Figure 1 the outer casing 1 terminates in a casing nozzle 19 adapted to receive a syringe needle, not shown. The nozzle 19 is covered by a sealing cap 21.

A limit ring 23 on the outer periphery of the cylinder 6 limits the outward stroke of the cylinder 6.

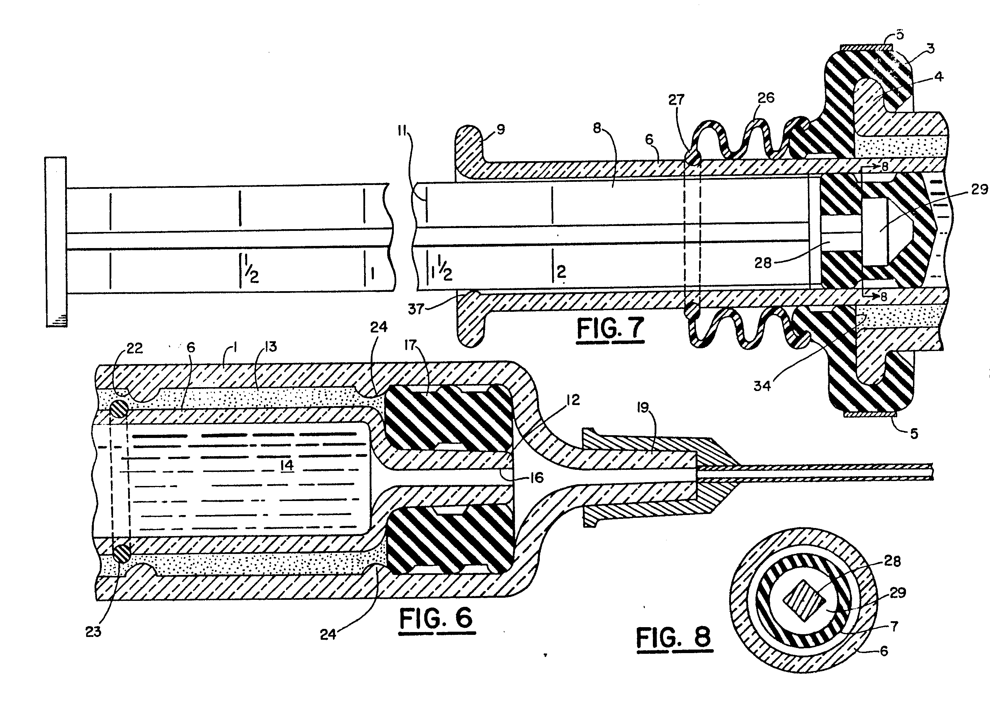

Figures 1 and 8 show an angular neck 28 under a securing head 29 on the end of the stem 8 on which the plunger 7 is mounted so that the stem does not rotate in the plunger 7. Figure 8 is actually a section of the modification of Figure 7, described hereafter, but the construction of this part is the same.

The tip of the nozzle 12 has cross grooves 18 in its end face which facilitate suction of material into the inner chamber.

The exact location of the tip of the cylinder nozzle 12 is accomplished by spacing the limit ring 23 on the cylinder 6 from the inside face 34 of the outer seal 3 at a distance slightly longer than the length of the cylinder nozzle 12 inserted into the passage of the nozzle gland 17 so that as the cylinder 6 is withdrawn from the outer casing 1, it is stopped when the tip of the cylinder nozzle is properly located relatively to the inner face 31 and the grooves 32 of the nozzle gland 17. In this form the outer seal 3 also extends over the cylinder 6 to a distance longer than the portion of the nozzle 12 in the passage of the nozzle gland 17 to protect against contamination. A spring stop 36 forms part of the stem 8 in such position that when depressed it remains concealed in the stem 8 during the mixing operation, but when the plunger is pulled out to abut an abutment 37 to suck into the cylinder all the mixture, then the spring stop 36 is freed and expands to prevent suction in the outer chamber from drawing the plunger inwardly while rejamming the cylinder 6. The spring stop 36 is depressed for insertion into the cylinder when the mixture is to be expelled through the needle.

In operation, first the unit is assembled. One way of assembling is herein described. The cylinder 6 is in the outer casing 1 with the nozzle 12 in the gland 17 the nozzle and the slit 39 being plugged by the plug 40. One of the required ingredients (such as liquid in Figure 1) is introduced into the outer chamber 13. Then the outer seal 3 is sprung over the casing flange 4 and over the cylinder 6. The other ingredient (such as a powder in Figure 1) is then introduced into the cylinder 6. Finally, the deformable plunger 7 is pushed over the abutment 37 into the cylinder 6 up to the spring stop 36 of the stem 8. During this insertion a small tube is inserted along the plunger side to vent the air from the inner chamber. The unit is thus hermetically sealed and the sterility of the interior moving parts and of the components in the chambers is preserved and accidental intermixture is positively prevented during storage.

In use, the cylinder 6 is pulled outwardly until the limit ring 23 abuts the inner face 34 of the outer seal 3, thereby locating the discharge tip of the cylinder 12 at the proper clearance relative to the grooves 32. Then the spring stop 36 is depressed into the stem 8 and plunger 7 is reciprocated repeatedly, alternately to expel the component from the inner chamber 14 into the outer chamber 13 and then to suck the mixed components back into the inner chamber 14 until the components are completely intermixed. The outer periphery of the nozzle 12 is of smaller diameter than the outside diameter of the cylinder 6. Therefore, the withdrawing of the nozzle 12 into the outer chamber 13 enlarges the capacity of the outer chamber and creates a suction effect which assists in expelling the contents from the inner chamber by the plunger 7. All the mixture is then sucked into the inner chamber 14 as the plunger 7 is pulled back to the abutment 37 and the spring stop 36 expands into blocking position to hold the plunger in position and prevent the suction in the empty outer chamber from drawing the plunger and the mixture inwardly while the nozzle 12 is jammed into the passage of the nozzle gland 17 by pushing cylinder 6 inwardly.

Finally, the cap 21 is removed and the needle or other member is placed on the casing nozzle 19, the spring stop 36 is depressed, the plunger 7 is pushed to expel the mixture through the needle with comparatively great force, depending on the rate of advance of the plunger per second and on the diameter of the tip of the nozzle 12.

In the embodiment shown in Figures 6 and 7, the nozzle gland 17 lacks the lips 38, but may be constructed as shown in Figure 12 if desired. The outer seal 3 is provided with a bellows-like sleeve 26, which terminates in a ring 27, which engages in a groove in the outer periphery of the cylinder 6. This hermetically seals the working portions of the outer surface of the cylinder 6 against contamination.

Figure 6 illustrates how a needle may be attached to the outer casing nozzle 19. In Figure 6 powder is provided in the outer chamber and liquid in the inner chamber.

The modification of Figures 9 and 10 lacks the casing nozzle 19. The gland 17 is modified and sealing engages with the casing and the nozzle 16 is longer and projects beyond the gland 17 and beyond the casing 1.

8

The nozzle is protected by a cap 21 which engages with both the nozzle and the gland 17.

The outer seal 3 is also of modified form and is held in place by an annular bead on the casing instead of by a flange.

In this case, a needle is attached to the nozzle 12 in use.

CLAIMS

1. A compartmental syringe comprising an outer casing, a cylinder reciprocable within the outer casing and spaced from the inner periphery of the outer casing to define an outer chamber, the interior of the said cylinder defining an inner chamber, sealing means at one end of the outer casing, said cylinder extending through said sealing means, a plunger working in the cylinder, a discharge nozzle on the inner end of the cylinder within said outer casing, nozzle sealing means normally sealing said discharge nozzle from the outer chamber, said nozzle sealing means unsealing said nozzle when withdrawn from said nozzle sealing means so as to communicate said inner chamber and outer chamber, whereby the working of the plunger intermixes the contents of the inner and outer chambers and finally sucks the mixture into the inner chamber.

2. A compartmental syringe according to claim 1, characterised by communicating means adjacent the discharge nozzle of the said cylinder for communicating the discharge from said cylinder with a syringe needle.

3. A compartmental syringe according to claim 2 wherein said communicating includes a nozzle projection on the outer casing adapted to receive an applicator.

4. A compartmental syringe according to claim 1 characterised in that the discharge nozzle projects through the nozzle sealing means, and is adapted to receive an applicator.

5. A compartmental syringe according to any preceding claim characterised by coacting means on the cylinder and on the outer casing to limit the stroke of the cylinder away from the sealing means so as to locate the tip of the discharge nozzle at a predetermined clearance from the nozzle sealing means.

6. A compartmental syringe according to any preceding claim characterised in that the capacity of the outer chamber is substantially equal to that of the inner chamber the outer diameter of the discharge nozzle being smaller than the outer diameter of the cylinder whereby the withdrawal of the cylinder from the nozzle sealing means creates a suction effect facilitating intermixture by the working of the plunger.

7. A compartmental syringe according to any preceding claim characterised in that the nozzle sealing means has an end surface  facing said cylinder and provided with deflecting means for imparting a whirling effect to the material discharge from the said discharge nozzle for intermixture with the material in the outer chamber.

8. A compartmental syringe according to any preceding claim characterised in that said nozzle sealing means is a gland having a passage therethrough fitting said discharge nozzle, and a releasable obstruction in said passage inhibiting discharge through said passage until the force of said discharge exceeds a predetermined pressure.

9. A compartmental syringe according to any preceding claim, characterised by means in the casing engaging the nozzle sealing means so as to resist movement of the nozzle sealing means with the nozzle during the withdrawal of the nozzle from said nozzle sealing means.

10. A compartmental syringe according to any preceding claim characterised by guiding means in the outer casing inhibiting tilting of said cylinder in said casing.

11. A compartmental syringe according to any preceding claim characterised by manipulating means connected to the plunger, and including releasable stop means to prevent inward movement of the plunger from its outward position in response to suction from the outer chamber.

12. A compartmental syringe according to claim 11 characterised in that the manipulating means comprises a manipulating stem extending from the plunger and the releasable stop means is resiliently retracted into the cylinder during manipulation and projecting into blocking position relatively to the cylinder in the outermost position of the plunger, thereby to prevent inward drawing of the plunger from its outermost position by suction in the chambers during reinsertion of the discharge nozzle into the nozzle sealing means.

13.    A compartmental syringe according to any preceding claim, characterised by a casing nozzle on the casing, a cap closing the casing nozzle and a plug extending from the cap into the casing nozzle and into the discharge nozzle obstructing accidental discharge therefrom.

14.    A compartmental syringe according to any preceding claim characterised by an outward extension of the sealing means ,over the cylinder beyond the casing, the extension covering the cylinder to a distance in excess of the stroke of movement of the cylinder for the withdrawal of the discharge nozzle from the nozzle sealing means.

FIG. 5

FIG. 2

FIG. 3    FIG. 4

FIG. 1

1/3

0005939

FIG. 7

FIG. 6

FIG. 8

2/3

0005939

FIG. 10

FIG. 9

FIG. 12

FIG. 11

FIG. 13

European Patent
Office

**EUROPEAN SEARCH REPORT**

0005939

Application number

EP 79 30 0854

| DOCUMENTS CONSIDERED TO BE RELEVANT | | | CLASSIFICATION OF THE APPLICATION (Int Cl²) |
|---|---|---|---|
| Category | Citation of document with indication, where appropriate, of relevant passages | Relevant to claim | |
| | US - A - 3 735 900 (GORES) <br> * Column 3, lines 35-68; column 4; column 5, lines 1-22; figures 1 to 3 * <br><br> -- <br><br> US - A - 3 678 931 (COHEN) <br> * Claim 1; figures * <br><br> -- | 1,5 <br><br><br><br><br> 1 | A 61 M 5/28 |
| A | US - A - 3 108 591 (KOLBAS) <br> * Claim 1; figures * <br><br> ---- | 1 | TECHNICAL FIELDS SEARCHED (Int.Cl.²) <br><br> A 61 M |
| | | | CATEGORY OF CITED DOCUMENTS <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |
| | The present search report has been drawn up for all claims | | &: member of the same patent family, corresponding document |
| Place of search <br> The Hague | Date of completion of the search <br> 07-09-1979 | Examiner <br> VANRUNXT | |

EPO Form 1503.1   06.78